# EUROPEAN PATENT SPECIFICATION

(11) **EP 0 713 403 B1**
(45) Date of publication and mention of the grant of the patent: **01.12.1999**
(21) Application number: 94926139.0
(22) Date of filing: 02.08.1994
(51) Int. Cl.: A61M 5/315

(54) **DOSING SYRINGE**
DOSIERSPRITZE
SERINGUE DOSEUSE

(30) Priority: 11.08.1993 GB 9316692
(43) Date of publication of application: 29.05.1996
(73) Proprietor: SMITHKLINE BEECHAM PLC, Brentford, Middlesex TW8 9EP (GB)
(72) Inventor: FLEWITT, Harry, Brentford Middlesex TW8 9BD (GB)
(74) Representative: Walker, Ralph Francis, Dr.
(86) International application number: EP9402554
(87) International publication number: WO9504563

(56) References cited:
- WO-A-91/13689
- DE-A- 4 134 911
- FR-A- 711 644
- FR-A- 2 625 981
- US-A- 4 112 945

## Description

This invention relates to a novel device, being a syringe for dispensing liquids, in particular liquid pharmaceutical formulations.

It is often necessary to administer liquid pharmaceutical formulations in accurate volumes, so as to strike a balance between administration of insufficient of the formulation to achieve the desired therapeutic effect, and administration of too much of the formulation so that undesirable side effects occur.

Liquid pharmaceutical formulations are often administered by means of a syringe capable of administering a set volume of the liquid. Such syringes may have a nozzle which can be fitted with a hypodermic needle for transdermal administration, or may be used for oral administration of the formulation to small children, babies etc who may have difficulty in swallowing a solid formulation form such as a tablet.

Such syringes include a cylinder and a piston slideably moveable within the cylinder, the volume of the cylinder and the distance the piston can move along the cylinder defining the volume which is administered by the syringe. Some such syringes are adjustable so that they can be adjusted to administer a preselected set volume, which can be selected in accordance with for example the seriousness of the disease to be treated and/or the age or weight of a child to which the formulation is to be administered.

Various constructions of such syringes are known, for example in EP 0154593, EP 0268191, EP 0293572, EP 0327910, EP 0338806, EP 0368191, EP 0373321, US 4112945, EP 0106578 and FR 711644. WO 91/13689 discloses a syringe-like applicator in which a plurality of partial strokes can be executed sequentially and releasably limited at the corresponding end by an end stop. The applicator of WO 91/13689 has no facility for locking the applicator to deliver a pre-set dose.

Generally such syringes suffer from excessive mechanical complexity, which leads to expense, the increased possibility of mechanical malfunction, and problems of patient compliance or confusion over the correct way to use the syringe. Moreover these syringes suffer from the problem of ensuring that once the syringe is set to administer a set dose, the syringe is not adjusted to administer an incorrect dose.

It is an object of this invention to provide an improved adjustable syringe that to some extent at least alleviates these problems. In particular it is an object of this invention to provide an adjustable syringe that can be easily set to a required dose, but thereafter is relatively difficult to adjust, so that once set, for example by a pharmacist, it is relatively difficult for a patient to vary the dose set, so that misuse of the syringe is reduced. Other objects of this invention will be apparent from the following description.

According to this invention a syringe suitable for administering liquids comprises a barrel which defines or comprises a cylinder, the cylinder having a nozzle at one end and including a piston which is longitudinally slideable within the cylinder, the distance the piston can move within the cylinder defining the volume which is administered by the syringe, the piston being operatively coupled to a slide which is arranged to move in parallel with the piston, the longitudinal distance the piston can slide within the cylinder being defined by the limits of travel of a stop surface on the barrel or the slide relative to end-stops on respectively the slide or barrel, *characterised* by the syringe being lockable into a state in which the stop surface and end stops can only move relative to each other within limits of travel selected from two or more selectable predetermined limits of travel.

The term "lockable" as used herein means that the limit of travel is easily selectable but once selected is locked such that the limit of travel cannot be changed, or is only changeable with considerable difficulty, or with damage to the syringe, or with leaving an indication by damage of the attempt to vary the limit of travel.

The cylinder may be defined by a cylindrical bore within the barrel, in which case the barrel and cylinder may be integral, Alternatively the barrel may be in the form of a sleeve wholly or partly surrounding the cylinder. In this latter case the barrel and cylinder may be separately made, and fastened together, enabling the barrel and cylinder to be made of different materials.

The barrel and slide are suitably in the form of sleeves surrounding each other and longitudinally slideable relative to each other. Either the slide may surround the barrel or the barrel may surround the slide, and in the latter option the slide may surround the cylinder in the form of a surrounding sleeve. The slide, barrel and/or cylinder may have cooperating guides, such as interlocking shaped parts etc to encourage linear relative longitudinal sliding, in a generally known manner, and/or may be of a cross section that encourages linear longitudinal relative sliding and restricts unintended relative rotation or lateral movement. Suitably the slide and barrel may be in the form of a sleeves wholly or partly enclosing each other and closely conforming to the cross section of each other and longitudinally slideable relative thereto in a telescoping manner and enclosing the cylinder.

The operative coupling between the piston and the slide may comprise a rigid linking, which may be releasable, for example by snap-fit cooperating elements on the piston and slide, or alternatively the piston and slide may be integral, The piston may include conventional features such as sealing rings etc.

In one embodiment of the invention the preselectable limits of travel may be selected by a construction of the syringe in which the stop surface comprises an insert which is insertable into a hole in the barrel of slide so as to project therefrom and to be removeable therefrom only with difficulty or by the use of a special tool, the projecting part of the insert forming a stop surface, the location of each such hole relative to the end stops defining one of the preselected travel distances.

The preselectable limits of travel may suitably be defined by the limits of travel of a stop surface such as a locking insert on the barrel or slide between end-stops defined as the ends of a generally longitudinal slot on respectively the slide or the barrel.

The syringe may include two or more such slots being of different lengths corresponding to the two or more preselectable limits of travel. Alternatively the slot may be defined by a gate having a ramped or stepped shape, each pan of the ramp or each step defining a different slot length. Alternatively the slot may be in the form of a gate within which the longitudinal position of the stop surface relative to the ends of the slot may be selected.

Alternatively the preselectable distances may be defined by the limits of travel of a stop surface on the barrel or slide between end stops defined by the positions of one or more projecting abutments on respectively the slide or the barrel.

The syringe may include pairs of stop surfaces and end-stops defining parallel travel distances of the same length to encourage smooth relative sliding.

The stop surface may comprise a pin insertable in a hole in the barrel or slide so as to project therefrom, the projecting part forming a stop surface, the location of each such hole relative to the end-stops defining one of the preselected travel distances.

In one form of the syringe which uses such a locking insert, the slide may be in the form of a sleeve wholly or partly enclosing the barrel, the sleeve having two or more holes passing completely through its wall, each hole being adjacent to a slot or a gate formed in or through the surface of the barrel, each hole defining a selectable position of the pin relative to the ends of the slot or gate, so as to define different limits of travel.

By insertion of the pin through one of the holes so that it projects beyond the surface of the sleeve on its inside and forms a projection, the distance the barrel and sleeve can slide relative to each other is defined by the travel of the projection acting as a stop surface within the corresponding slot or the gate, the longitudinal ends of which act as end stops. In one version of this embodiment the pin is in the form of a key which will only fit one of the holes.

In another form of the syringe which uses such a pin, the slide may be in the form of a sleeve wholly or partly enclosing the barrel, the sleeve having two or more slots, each slot being of a different length, or a gate, passing completely through its wall, the corresponding area of the surface of the barrel having one or more holes therein, each hole being adjacent to a slot or to the gate each hole defining a selectable position of the pin relative to the ends of the slot or gate, so as to define different limits of travel.

By insertion of a locking insert through a slot or the gate into one of the holes such that it projects, to form a stop surface, from the surface of the barrel into the slot or gate, the longitudinal ends of which act as end stops, the limit of travel of the sleeve and barrel relative to each other is defined by the travel of the projection within the corresponding slot or the gate. Each hole thereby defines a selectable position of the locking insert relative to the ends of the slot or gate, so as to define different limits of travel. In one version of this embodiment the locking insert is in the form of a key which will only fit one of the holes.

In a second embodiment, the slide is in the form of a sleeve which surrounds the cylinder and is itself surrounded by the barrel, and is rotatable relative to the barrel as well as being longitudinally slideable relative to the cylinder. Relative rotation of the slide and the barrel alters the relative positions of the stop surface and the end stops so as to define different limits of travel of the stop surface within slot(s) or a gate, and the slot(s) or gate are moveable relative to a fixed stop surface on the barrel or slide, and the slide and barrel are lockable in position by means of a locking insert which is insertable into a hole passing through the wall of the barrel into a corresponding hole in the slide and is removeable therefrom only with difficulty or the use of a special tool.

The barrel may have a stepped gate passing completely through its wall, with a projection on the surface of the slide from the area of the slide adjacent to the gate, and relative rotation of the barrel on the slide may bring the projection into position in line with pans of the gate having different lengths so as to define different limits of travel.

The locking insert may, as above, be in the form of a key insertable into any one locking position so as to prevent incorrect administration. Similarly the locking insert may cooperate with a fail-safe mechanism as described above.

In a third embodiment the gate or slot(s) and stop surface are moveable relative to each other, and the stop surface is capable of being snap-fitted into position within the slot or gate against the resilience of the syringe material in the region of the slot or gate, the stop surface and/or the region having ramped surfaces such that when snap fitted into the slot or gate the stop surface is locked within a selected slot or pan of the gate so as to define a limit of travel.

In this embodiment the stop surface may suitably be a projection from the barrel or slide, having a wedge faced profile which enables the projection to move across the surface of the slide or barrel respectively as the syringe is assembled and to lockably snap into position in the selected slot or part of this gate and having an abuttment face opposite the wedge face to prevent the stop surface from thereafter being removed.
The locking insert, as mentioned above, may comprise a key which is capable of fitting into only one of two or more positions, e.g. holes, in the barrel or slide, to prevent inadvertent insertion into the wrong position which might result in administration of an incorrect volume from the syringe. Such a key may also be so shaped that once fitted into position it cannot be removed, or only with difficulty or the use of a special tool, so as to prevent removal and reinsertion into the incorrect position. This may be achieved for example by a key which is a tight snap fit within a corresponding receptacle or hole on the syringe, or by a key which fits substantially flush with the surface of the syringe to make removal difficult.

Such a key may also be arranged to co-operate with a fail-safe mechanism which only permits the syringe to work when such a pin is in place. For example the insertion of the pin into the hole may disengage a locking mechanism which prevents the slide from sliding relative to the barrel.

The syringe of the invention may be easily pre-set by a pharmacist or by the user to deliver only a preselected prescribed dose of a liquid pharmaceutical formulation.

Suitably the entire assembly of barrel, cylinder, piston and slide may be made of conventional plastics materials, or the cylinder may be made separately of other conventional inert materials such as glass. The relatively simple construction of the syringe of the invention, and the avoidance of complex mechanisms enables the syringe to be moulded in "friendly" shapes, avoiding the more "clinical" appearance of known syringes, making them particularly suitable for use with small children.

The invention will now be described by non-limiting examples, with reference to the following figures which show:
- Figs. 1 to 4.: A syringe of the invention in section and dissembled views, and keys for the syringe.
- Figs. 5 to 7.: A syringe of the invention in section and dissembled views, and keys for the syringe.
- Figs. 8 to 11: Syringes of the invention in section and assembled views.
- Figs. 12 to 14: A syringe of the invention in section and assembled views.
- Figs. 15 to 17: A syringe of the invention in section and assembled views.
- Figs. 18 and 19: A syringe of the invention in section and assembled view.

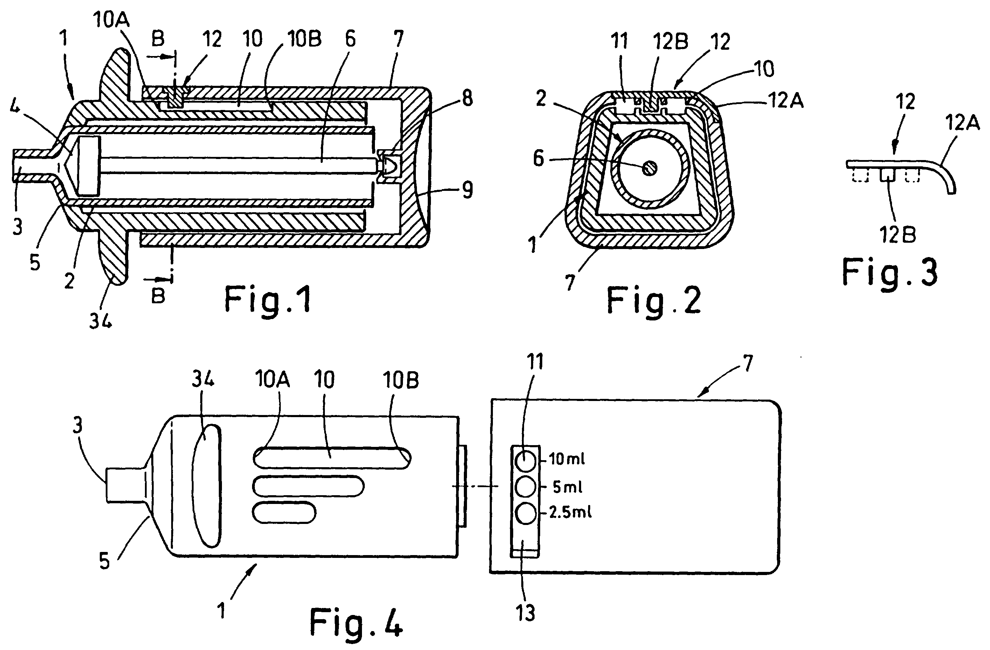

In Fig. 1 a syringe of the invention is shown in longitudinal section. In Fig. 2 the syringe of Fig. 1 is shown in cross section about the line B----B of Fig. 1. In Fig. 4, the syringe of Fig. 1 is shown in a semi-dismantled view. The syringe comprises a barrel (1) in the form of a sleeve which surrounds a cylinder (2) having a nozzle (3) at one end. Within the cylinder (2) there is a piston (4) which is longitudinally slideable within the cylinder (2). The cylinder (2) is retained within the barrel (1) by a snap fit connection at (5). The nozzle (3) may for example be shaped to receive a hypodermic needle, be so shaped as to receive a teat (for small babies to suck) or be a simple nozzle, as shown, to allow a volume to be dispensed.

The piston (4) is operatively coupled by a rigid link rod (6) to a slide (7) in the form of a sleeve which surrounds and is longitudinally slideable on the barrel (1). The link rod (6) fits into a snap-fit male-female socket (8) in the end wall (9) of slide (7). As shown in Fig 2, the cross section of the sleeve (7) corresponds closely to that of the barrel (2), both being of a substantially rectangular cross section to inhibit relative rotation. The relative clearances of the parts shown in Figs. 1 to 6 are exaggerated for clarity.

In the outer surface of barrel (1) are a number of slots (10), of differing length. Passing completely through the wall of the slide (7) are holes (11), corresponding in number to the number of slots (10), and located such that when the sleeve (7) is in place on the barrel (1) each hole (11) is adjacent to a slot (10).

In Figs. 1 and 2 a pin (12) is shown inserted completely through a hole (11) so as to extend internally beyond the inner wall of slide (7) to form a projection into the interior of the slide, into the slot (10). The distance over which the slide (7) can slide relative to the barrel (1) is consequently defined by the limits of travel of the inwardly projecting pan of pin (12) acting as a stop surface between the ends (10A, 10B) of slot (10) acting as end stops. It will be apparent that insertion of the pin (12) into each of the holes (11) will result in a different limit of travel of the pin (12) in a slot (10).

With reference to Fig. 3 a pin (12) in the form of a key is shown in more detail. The pin (12) is in the form of a strip (12A) of plastics material conforming in shape to the section of the slide (7). This strip (12A) is shaped to fit flush into a recess (13) in the outer surface of slide (7) and to be retained therein by a snap-fit or friction fit action, thereby making it easy to insert but difficult to remove. From the underside of the strip (12A) projects a pin (12B). The position of the pin (12B) is such that it will only project through one of the holes (11) in the slide (7). The possible positions of pins (12B) on alternative strips (12A) which could be inserted through other holes (11) is shown by dotted lines in Fig. 2.

In Fig. 4 the syringe of Figs 1 and 2 is shown in a partly dissembled view showing the relationship of the barrel (1) and slide (7) before assembly.

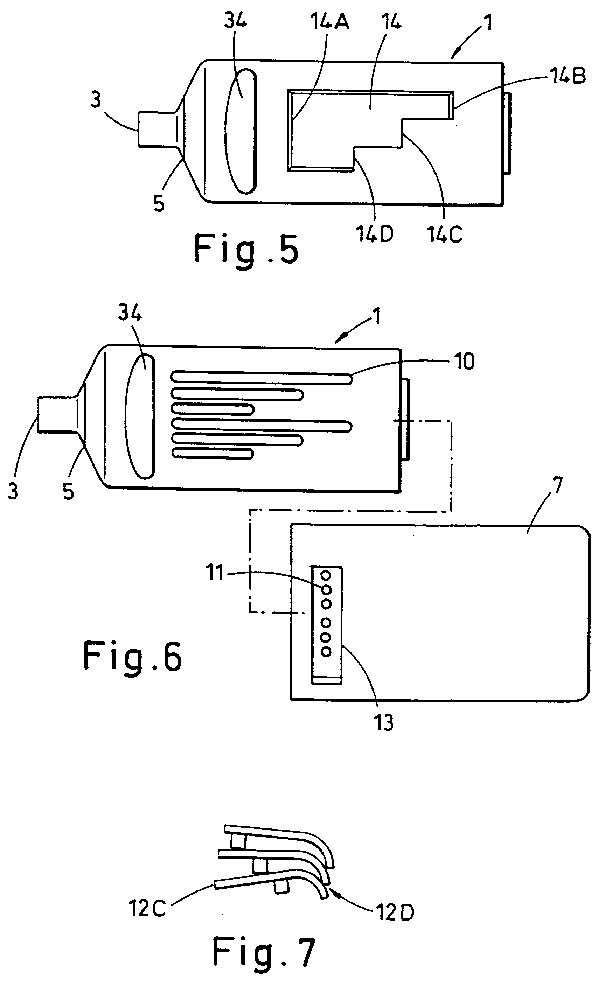

In Figs. 5 and 6, alternate arrangements of the barrel (1) are shown. In Fig. 5 a barrel (1) has a stepped gate (14) formed in its surface. The distance between the end (14A) and each step (14B, 14C, 14D) acting as end stops defines the limits of travel of stop surface in the form of a pin (12) when the slide (7) as shown in Fig 3 is in place on barrel (1).

In Fig 6 a barrel (1) is shown in which slots (10) are formed in a manner similar to that of Fig. 1. In the barrel of Fig. 6 two sets of slots (10) are provided, so that pairs of slots (10) of the same length are present in the surface of the barrel (1). A pin in the form of a key (not shown) similar to (12A) of Fig 3 is again provided which can project through holes (11) passing through the wall of slide (7) in the same manner as the pin (12A) of Fig. 1, but having two pins (12B) each capable of projecting trough a different hole (11) corresponding to a pair of slots (10).

Fig. 7 shows how three keys (12C) may be moulded integrally linked at thinned points (12D) at which they can be broken apart and separated. The integral keys (12C) may be provided together with a syringe, for example fitting into a corresponding shaped receiving cavity in the syringe body.

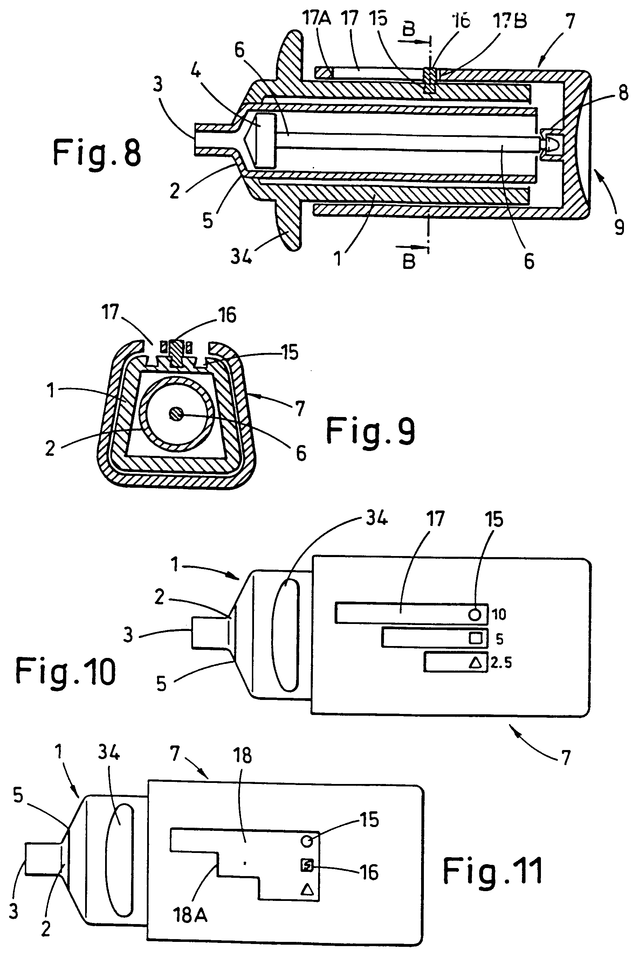

Referring to Figs. 8 to 11, an alternative arrangement of syringe is shown in longitudinal section in Fig. 8, in a cross section in Fig. 9 about the line B......B of Fig 8, and in an assembled view in Figs 10 and 11. The syringe comprises a cylinder (2), piston (4) and link rod (6) snap-fitted within a barrel (1) in a similar arrangement to that shown in Fig. 1. The syringe of Fig. 8 has a barrel (1) and sleeve-form slide (7) similar in shape to that of Fig. 1. Again the clearance between the parts shown in Fig. 8 to 11 is exaggerated for clarity.

In the syringe of Figs 8 to 11, the barrel (1) is provided with a number of socket holes (15) each capable of receiving an insertable pin (16) and retaining the pin by a snap-fit action. The syringe is provided with a number of pins (16) each of a cross section, e.g. circular, square or triangular which allows it to be inserted only in a selected hole (15) of corresponding shape, as shown in Figs 10 and 11. The pin (16) projects from the surface of the barrel (1) to form a projection which acts as a stop surface.

As shown in Fig. 8, the cross section of the barrel (1) and the slide sleeve (7) are substantially rectangular, so as to minimise relative axial rotation.

In the slide (7) is formed either a number of slots of different lengths (17) as shown in Fig 10 or a stepped gate (18) as shown in Fig 11, both in the form of an aperture passing completely through the wall of the slide (7). With the pin (16) in place in a socket (15) and the slide (7) in place on the barrel (17), the distance that the slide can slide relative to the barrel (1) is defined by the limit of travel of the pin (16) between the ends (17A, 17B), (18A, 18B) of the slot (17) or gate (18) acting as end stops. The socket holes (15) and pin (16) are distinctively co-operatively shaped so that the pin (16) can only be inserted in the socket for which it is intended.

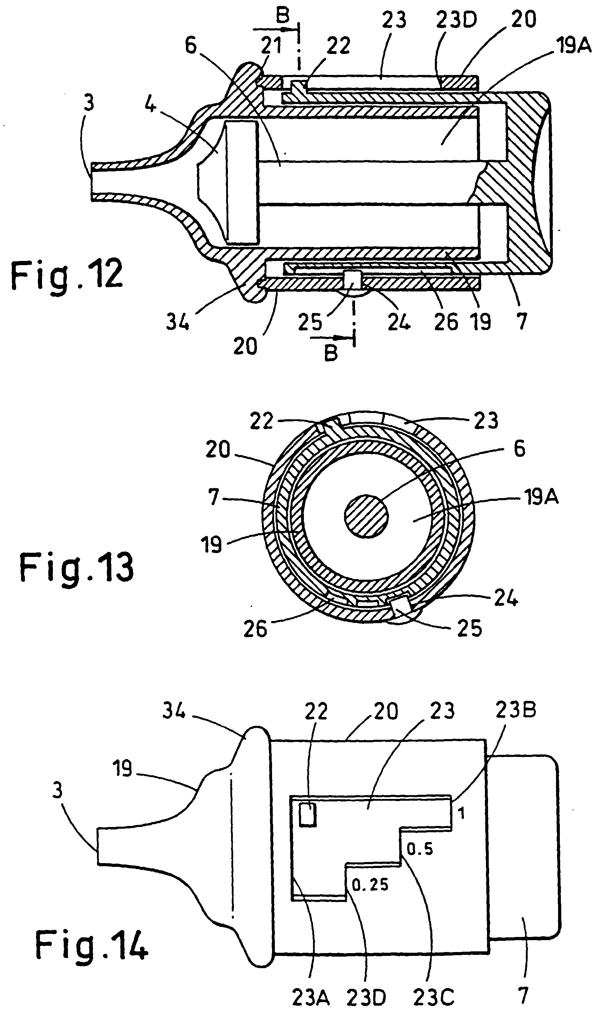

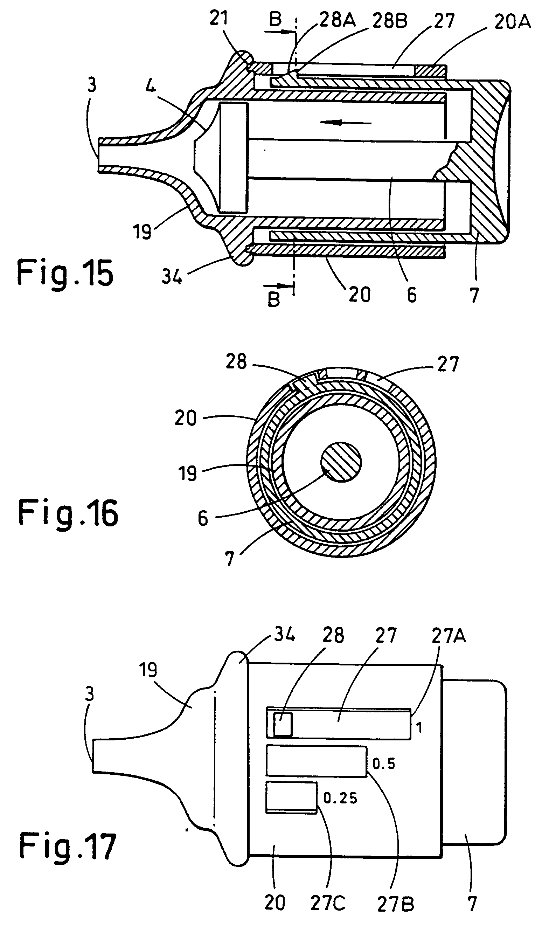

Referring to Figs. 12 to 14, a further alternative arrangement of syringe is shown in longitudinal section in Fig. 12, in a cross sectional part view about the line B----B in Fig. 13, and assembled in Fig. 14. The clearance between the parts shown in Figs. 12 to 14 is exaggerated for clarity.

The syringe comprises a cylinder (19). Within the cylinder (19) is a piston (4) which is integrally formed with a link rod (6) and slide (7). The cylinder (19) is surrounded by a barrel (20) in the manner of a sleeve, and the slide (7) is itself partly surrounded by the barrel (20) in the manner of a slideable sleeve surrounding the cylinder (19) and itself surrounded by the barrel (20). In the region of surround the slide (7), cylinder (19) and barrel (20) are of a cylindrical shape so that the slide (7) may be rotated axially on the cylinder (19) in addition to being longitudinally slideable thereon. The barrel (20) is fitted onto the cylinder (19) by a friction fit, or screw or snap fit connection at (21). On the outer wall of the slide (7) is an integral projection (22). In the barrel (20) is a stepped gate (23) extending completely through the wall of the barrel (20). The projection (22) projects into the gate (23) so that the distance the slide (7) can slide relative to the barrel (20) is defined by the travel of the projection (22) acting as a stop surface between the ends (23A, 23B, 23C, 23D) of the gate (23) acting as end stops.

The slide (7) and barrel (20) may be relatively rotated so that the projection (22) is adjacent to a selected one of the ends (23B, 23C, 23D) of the steps of the gate (23). In the wall of barrel (20) is a hole (24), through which may be inserted a locking pin (25), which cooperates wit one of a number of guide grooves (26) formed in the wall of the slide (7), so that the slide (7) is locked into a position adjacent to one of the stepped ends (23B, 23C, 23D).

Referring to Figs. 15 to 17, a modification of the syringe of Figs. 12 to 14 is shown, in longitudinal section in Fig. 15, in a cross section about the line B--B in Fig. 16, and assembled in Fig. 17. The clearance between the parts shown in Fig. 15 to 17 is exaggerated for clarity.

The arrangement of the syringe of Fig. 15 to 17 is similar to that of Fig. 12 to 14, having a cylinder (19) and barrel (20), a slide and integral piston and link rod (7), (4), (6), the barrel (20), fitting to the cylinder (19) by a snap fit, friction fit or screw fit at (21).

In the barrel (20) are a number of slots (27) of different lengths. On the slide (7) is an integral projection (28), which is of a wedge shape. The syringe may be assembled by relatively rotating the unassembled slide (7) and barrel (20) such that the projection (28) is aligned with a selected one of the slots (27), then inserting the slide (7) into the barrel (20) in the longitudinal direction shown by the arrow so that the wedge face (28A) of the projection (28) snaps under the region (20A) of the barrel (20) adjacent to a selected one of the slots (27), and then into the space within the slot (27). The opposite abutment face (28B) of the projection (28) then prevents its removal from the slot (27). The distance the slide (7) and barrel (20) can slide relative to each other is then defined by the travel of the projection (28) acting as a stop surface between the ends (27A, 27B), acting as end stops, of the slot in which it lies.

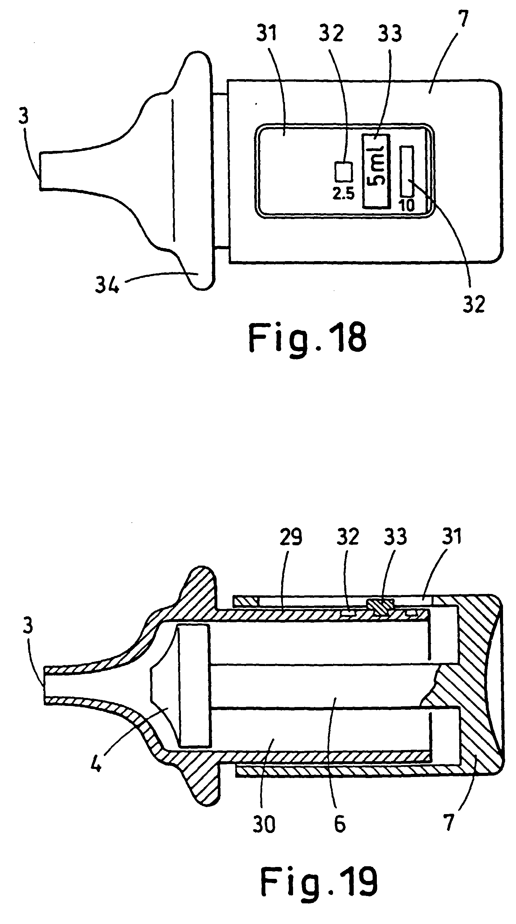

Referring to Figs 18 and 19, a syringe is shown in an assembled view in Fig 18, and in a longitudinal section perpendicular to the plane of Fig 18 in Fig 19. The syringe comprises a barrel (29), with an integral cylinder defined by the cylindrical bore (30) within the barrel (29). Within cylinder (30) is a piston (4) integrally linked to a slide (7) in the form of a sleeve surrounding the barrel (29) and longitudinally slideable thereon.

In slide (7) is a longitudinal slot (31) passing completely through the wall of the slide (7). In the wall of barrel (29) are holes (32) into which can be fitted pins (33). Each of the holes (32) is at a different longitudinal distance along the slot (31) corresponding to the administration of a preselected volume of liquid from the syringe. Each of the holes (32) is shaped so as to receive, in a tight snap fit fitting, a corresponding pin (33) which once fitted cannot be easily removed. The limit of longitudinal travel of the pin (33) acting as a stop surface between the end stops defined by the ends of the slot (31) defines the volume of liquid dispensed. By selection and fitting of an appropriate pin (33) the volume administered can be selected. In Figs 18 and 19 the clearance between the slide pans is exaggerated for clarity.

The operation of the syringes of Figs. 1 to 19 is similar. With the piston (4) at the end of the cylinder (2), (19), (30) adjacent to the nozzle (3), the nozzle (3) is inserted in a liquid (not shown) and the piston is then drawn back by pulling the slide (7) to such liquid into the cylinder (2), (19), (30). The volume of liquid which can be drawn into the cylinder (2), (19), (30) is defined by the limits of travel of the projection (12), (16), (22), (23), (33) within the slot (10), (17), (27), (32) or gate (18), (23). The liquid may then be ejected from the syringe via the nozzle (3) in a conventional manner. Conveniently the slide (7) may be moved towards the nozzle (3) by the user's thumb pressure whilst the user's fingers grip the finger grips (34).

Each of the syringes of Figs 1 to 16 may be further provided with a holder for a pin (12), (16), (25) supplied with the syringe. Conveniently a pin (16), (25), (33) may be supplied as a nose pin which also fits the nozzle (3).

## Claims

1. A syringe suitable for administering liquids comprising a barrel (1) which defines or comprises a cylinder (2), the cylinder (2) having a nozzle (3) at one end and including a piston (4) which is longitudinally slideable within the cylinder (2), the distance the piston (4) can move within the cylinder (2) defining the volume which is administered by the syringe, the piston (4) being operatively coupled to a slide (7) which is arranged to move in parallel with the piston (4), the longitudinal distance the piston (4) can slide within the cylinder (2) being defined by the limits of travel of a stop surface (12) on the barrel (1) or the slide (7) relative to end-stops (10A, 10B) on respectively the slide (7) or barrel (1), *characterised* in that:
the syringe is lockable into a state in which the stop surface (12) and end stops (10A, 10B) can only move relative to each other within limits of travel selected from two or more selectable predetermined limits of travel, and in that the stop surface (12) comprises an insert (12A, 12B) which is insertable into a hole (11) in the barrel (1) or slide (7) so as to project therefrom and to be removeable therefrom only with difficulty or by the use of a special tool, the projecting part (12B) of the insert forming a stop surface (12), the location of each such hole (11) relative to the end-stops (10A, 10B) defining one of the preselected travel distances.

2. A syringe according to claim 1 *characterised* in that the preselectable limits of travel are defined by the limits of travel of a stop surface (12) on the barrel (1) or slide (7) between end-stops (10A, 10B) defined as the ends of a generally longitudinal slot (10) on respectively the slide (7) or the barrel (1).

3. A syringe according to claim 2 *characterised* in that the syringe includes two or more such slots (10) being of different lengths corresponding to the two or more preselectable limits of travel, or the slot (10) is defined by a gate (14) having a ramped or stepped shape, each part of the ramp or each step defining a different slot length, or the slot is in the form of a gate (18) within which the longitudinal position of the stop surface (16) relative to the ends of the slot may be selected.

4. A syringe according to claim 1 *characterised* in that the slide (7) is in the form of a sleeve wholly or partly enclosing the barrel (1), the sleeve having two or more holes (11) passing completely through its wall, each hole (11) being adjacent to a slot (10) or a gate (14) formed in or through the surface of the barrel (1), each hole (11) defining a selectable position of the insert (12) relative to the ends of the slot (10) or gate (14), so as to define different limits of travel.

5. A syringe according to claim 1 *characterised* in that the slide (7) is in the form of a sleeve wholly or partly enclosing the barrel (1), the sleeve (7) having two or more slots (17), each slot (17) being of a different length, or a gate (18), passing completely though its wall, the corresponding area of the surface of the barrel (1) having one or more holes (15) therein, each hole (15) being adjacent to a slot (17) or to the gate (18), each hole (15) defining a selectable position of the insert (16) relative to the ends of' the slot (17) or gate (18), so as to define different limits of travel.

6. A syringe suitable for administering liquids comprising a barrel (20) which defines or comprises a cylinder (19), the cylinder (19) having a nozzle (3) at one end and including a piston (4) which is longitudinally slideable within the cylinder (19), the distance the piston (4) can move within the cylinder (19) defining the volume which is administered by the syringe, the piston (4) being operatively coupled to a slide (7) which is arranged to move in parallel with the piston (4), the longitudinal distance the piston (4) can slide within the cylinder (19) being defined by the limits of travel of a stop surface (22) on the barrel (1) or the slide (7) relative to end-stops (23A, 23B) on respectively the slide (7) or barrel (1), *characterised* by;
the syringe being lockable into a state in which the stop surface (22) and end stops (23A, 23B) can only move relative to each other within limits of travel selected from two or more selectable predetermined limits of travel, the slide (7) being in the form of a sleeve which surrounds the cylinder (19) and is itself surrounded by the barrel (20), and is rotatable relative to the barrel (20), as well as being longitudinally slideable relative to the cylinder (19), and relative rotation of the slide (7) and the barrel (19) alters the relative positions of the stop surface (22) and the end stops (23A, 23B) so as to define different limits of travel of the stop surface within slot(s) or a gate (23), the slot(s) or gate (23) being moveable relative to a fixed stop surface (22) on the barrel (20) or slide (7), and the slide (7) and barrel (20) being lockable in position by means of a locking insert (25) which is insertable into a hole (24) passing through the wall of the barrel (20) into a corresponding hole in the slide (7) and is removeable therefrom only with difficulty or the use of a special tool.

7. A syringe according to claim 6 *characterised* by the barrel (20) having a stepped gate (23) passing completely through its wall, there being a projection (22) on the surface of the slide (7) from the area of the slide (7) adjacent to the gate (23), and relative rotation of the barrel (20) on the slide (7) brings the projection (22) into position in line with parts of the gate (23) having different lengths, so as to define different limits of travel.

8. A syringe suitable for administering liquids comprising a barrel (20) which defines or comprises a cylinder (19), the cylinder (19) having a nozzle (3) at one end and including a piston (4) which is longitudinally slideable within the cylinder (2), the distance the piston (4) can move within the cylinder (19) defining the volume which is administered by the syringe, the piston (4) being operatively coupled to a slide (7) which is arranged to move in parallel with the piston (4), the longitudinal distance the piston (4) can slide within the cylinder (19) being defined by the limits of travel of a stop surface (28) on the barrel (20) or the slide (7) relative to end-stops (27A, 27B) on respectively the slide (7) or barrel (1) the end stops (27A, 27B) being defined as the limits of travel of the stop surface (28) within a gate or slot (27), *characterised* by;
the syringe being lockable into a state in which the stop surface (28) and end stops (27A, 27B) can only move relative to each other within limits of travel selected from two or more selectable predetermined limits of travel, the gate or slot(s) (27) and stop surface (28) being moveable relative to each other, and the stop surface (28) being capable of being snap-fitted into position within the slot or gate (27) against the resilience of the syringe material in the region of the slot or gate (27), the stop surface (28) and/or the region having ramped surfaces (28A) such that when snap fitted into the slot or gate (27) the stop surface (28) is locked within a selected slot or part of the gate (27) so as to define a limit of travel.

9. A syringe according to claim 8 *characterised* in that the stop surface (28) is a projection from the barrel or slide (7), having a wedge faced profile which enables the projection (28) to move across the surface of the slide or barrel (20) respectively as the syringe is assembled and to lockably snap into position in the selected slot or part of the gate (27), and having an abutment face (28B) opposite the wedge face (28A) to prevent the stop surface (28) from thereafter being removed.

10. A syringe according to claim 1 or 6 *characterised* in that the insert (12) comprises a key which is capable of fitting into only one of two or more positions (11) in the barrel (1) or slide (7).

## Patentansprüche

1. Spritze, die für die Verabreichung von Flüssigkeiten geeignet ist, mit einer Trommel (1), die einen Zylinder (2) definiert oder aufweist, wobei der Zylinder (2) eine Düse (3) an einem Ende aufweist und einen Kolben (4) einschließt, der in Längsrichtung innerhalb des Zylinders (2) gleitbar ist, wobei der Abstand, um den sich der Kolben (4) innerhalb des Zylinders (2) bewegen kann, das Volumen definiert, das durch die Spritze verabreicht wird, wobei der Kolben (4) treibend mit einem Schieber (7) gekoppelt ist, der derart angeordnet ist, daß er sich parallel zu dem Kolben (4) bewegt, wobei der Längsabstand, um den der Kolben (4) innerhalb des Zylinders (2) gleiten kann, durch die Bewegungsbegrenzungen einer Anschlagfläche (12) an der Trommel (1) oder dem Schieber (7) bezüglich Endanschlägen (10A, 10B) an dem Schieber (7) bzw. der Trommel (1) definiert ist,
dadurch **gekennzeichnet,** daß:
die Spritze in einen Zustand verriegelbar ist, in dem sich die Anschlagfläche (12) und die Endanschläge (10A, 10B) bezüglich einander nur innerhalb von Bewegungsbegrenzungen bewegen können, die aus zwei oder mehr wählbaren vorbestimmten Bewegungsbegrenzungen gewählt werden, und dadurch, daß die Anschlagfläche (12) einen Einsatz (12A, 12B) aufweist, der in eine Öffnung (11) in der Trommel (1) oder dem Schieber (7) derart einführbar ist, daß er von diesen vorsteht und von diesen nur mit Schwierigkeiten oder durch Verwendung eines speziellen Werkzeugs entfernbar ist, wobei der vorstehende Teil (12B) des Einsatzes eine Anschlagfläche (12) bildet, und wobei die Stelle einer jeden derartigen Öffnung (11) bezüglich der Endanschläge (10A, 10B) einen der vorgewählten Bewegungsabstände definiert.

2. Spritze nach Anspruch 1,
dadurch **gekennzeichnet,** daß
die vorauswählbaren Bewegungsbegrenzungen durch die Bewegungsbegrenzungen einer Anschlagfläche (12) an der Trommel (1) oder dem Schieber (7) zwischen Endanschlägen (10A, 10B) definiert sind, die als die Enden eines im allgemeinen in Längsrichtung ausgebildeten Schlitzes (10) an dem Schieber bzw. der Trommel (1) definiert sind.

3. Spritze nach Anspruch 2,
dadurch **gekennzeichnet,** daß
die Spritze zwei oder mehr derartige Schlitze (10) aufweist, die unterschiedliche Längen aufweisen und den zwei oder mehr vorwählbaren Bewegungsbegrenzungen entsprechen, oder daß der Schlitz (10) durch ein Tor (14) mit einer rampenförmigen oder gestuften Form definiert ist, wobei jeder Teil der Rampe oder jede Stufe eine unterschiedliche Schlitzlänge definiert, oder daß der Schlitz in Form eines Tores (18) ausgeführt ist, innerhalb dessen die Längsstellung der Anschlagfläche (16) bezüglich der Enden des Schlitzes ausgewählt werden kann.

4. Spritze nach Anspruch 1,
dadurch **gekennzeichnet,** daß
der Schieber (7) in Form einer Hülse ausgeführt ist, welche die Trommel (1) ganz oder teilweise umschließt, wobei die Hülse zwei oder mehrere Öffnungen (11) aufweist, die vollständig durch ihre Wand treten, wobei jede Öffnung an einen Schlitz (10) oder ein Tor (14) benachbart ist, der/das in oder durch die Oberfläche der Trommel (1) ausgebildet ist, und wobei jede Öffnung (11) eine wählbare Stellung des Einsatzes (12) bezüglich der Enden des Schlitzes (10) oder des Tores (14) definiert, so daß unterschiedliche Bewegungsbegrenzungen definiert werden.

5. Spritze nach Anspruch 1,
dadurch **gekennzeichnet,** daß
der Schieber (7) in Form einer Hülse ausgeführt ist, welche die Trommel (1) ganz oder teilweise umschließt, wobei die Hülse (7) zwei oder mehrere Schlitze (17), wobei jeder Schlitz (17) von einer unterschiedlichen Länge ist, oder ein Tor (18) aufweist, das vollständig durch ihre Wand tritt, wobei der entsprechende Oberflächenbereich der Trommel (1) eine oder mehrere Öffnungen (15) darin aufweist, wobei jede Öffnung (15) an einen Schlitz (17) oder an das Tor (18) benachbart ist, wobei jede Öffnung (15) eine wählbare Stellung des Einsatzes (16) bezüglich der Enden des Schlitzes (17) oder des Tores (18) definiert, so daß unterschiedliche Bewegungsbegrenzungen definiert werden.

6. Spritze, die für die Verabreichung von Flüssigkeiten geeignet ist, mit einer Trommel (20), die einen Zylinder (19) definiert oder aufweist, wobei der Zylinder (19) eine Düse (3) an einem Ende aufweist und einen Kolben (4) einschließt, der in Längsrichtung innerhalb des Zylinders (19) gleitbar ist, wobei der Abstand, um den sich der Kolben (4) innerhalb des Zylinders (19) bewegen kann, das Volumen definiert, das durch die Spritze verabreicht wird, wobei der Kolben (4) treibend mit einem Schieber (7) gekoppelt ist, der derart angeordnet ist, daß er sich parallel zu dem Kolben (4) bewegt, wobei der Längsabstand, um den der Kolben (4) innerhalb des Zylinders (19) gleiten kann, durch die Bewegungsbegrenzungen einer Anschlagfläche (22) an der Trommel (1) oder dem Schieber (7) bezüglich Endanschlägen (23A, 23B) an dem Schieber (7) bzw. der Trommel (1) definiert ist,
dadurch **gekennzeichnet,** daß
die Spritze in einen Zustand verriegelbar ist, in dem sich die Anschlagfläche (22) und die Endanschläge (23A, 23B) bezüglich einander nur innerhalb von Bewegungsbegrenzungen bewegen können, die aus zwei oder mehr wählbaren vorbestimmten Bewegungsbegrenzungen gewählt werden, wobei der Schieber (7) in Form einer Hülse ausgeführt ist, die den Zylinder (19) umgibt und selbst durch die Trommel (20) umgeben ist und bezüglich der Trommel (20) drehbar ist, sowie in Längsrichtung bezüglich des Zylinders (19) gleitbar ist, und wobei die Relativdrehung des Schiebers (7) und der Trommel (19) die Relativstellungen der Anschlagfläche (22) und der Endanschläge (23A, 23B) derart verändert, daß unterschiedliche Bewegungsbegrenzungen der Anschlagfläche innerhalb des Schlitzes (der Schlitze) oder eines Tores (23) definiert werden, wobei der oder die Schlitz(e) oder das Tor (23) bezüglich einer festen Anschlagfläche (22) an der Trommel (20) oder dem Schieber (7) beweglich sind, und der Schieber (7) und die Trommel (20) in einer Stellung mittels eines Verriegelungseinsatzes (25) verriegelbar sind, der in eine Öffnung (24), die durch die Wand der Trommel (20) tritt, in eine entsprechende Öffnung in dem Schieber (7) einführbar ist und von dieser nur mit Schwierigkeiten oder unter Verwendung eines speziellen Werkzeugs entfernbar ist.

7. Spritze nach Anspruch 6,
dadurch **gekennzeichnet,** daß
die Trommel (20) ein gestuftes Tor (23) aufweist, das vollständig durch deren Wand tritt, wobei ein Vorsprung (22) an der Oberfläche des Schiebers von dem Bereich des Schiebers (7), der an das Tor (23) benachbart ist, vorliegt, und eine Relativdrehung der Trommel (20) und des Schiebers (7) den Vorsprung (22) in eine Stellung in einer Linie mit Teilen des Tores (23) mit unterschiedlichen Längen bringt, so daß unterschiedliche Bewegungsbegrenzungen definiert werden.

8. Spritze, die für die Verabreichung von Flüssigkeiten geeignet ist, mit einer Trommel (20), die einen Zylinder (19) definiert oder aufweist, wobei der Zylinder (19) eine Düse (3) an einem Ende aufweist und einen Kolben (4) einschließt, der in Längsrichtung innerhalb des Zylinders (19) gleitbar ist, wobei der Abstand, um den sich der Kolben (4) innerhalb des Zylinders (19) bewegen kann, das Volumen definiert, das durch die Spritze verabreicht wird, wobei der Kolben (4) treibend mit einem Schieber (7) gekoppelt ist, der derart angeordnet ist, daß er sich parallel zu dem Kolben (4) bewegt, wobei der Längsabstand, um den der Kolben (4) innerhalb des Zylinders (19) gleiten kann, durch die Bewegungsbegrenzungen einer Anschlagfläche (28) an der Trommel (20) oder dem Schieber (7) bezüglich Endanschlägen (27A, 27B) an dem Schieber (7) bzw. der Trommel (1) definiert ist, wobei die Endanschläge (27A, 27B) als die Bewegungsbegrenzungen der Anschlagfläche (28) innerhalb eines Tores oder Schlitzes (27) definiert sind,
dadurch **gekennzeichnet,** daß:
die Spritze in einen Zustand verriegelbar ist, in dem sich die Anschlagfläche (28) und die Endanschläge (27A, 27B) bezüglich einander nur innerhalb von Bewegungsbegrenzungen bewegen können, die aus zwei oder mehr wählbaren vorbestimmten Bewegungsbegrenzungen gewählt werden, wobei das Tor oder der oder die Schlitz(e) (27) und die Anschlagfläche (28) bezüglich einander bewegbar sind, und die Anschlagfläche (28) in der Lage ist, an ihre Stelle innerhalb des Schlitzes oder des Tores (27) gegen die Nachgiebigkeit des Spritzenmaterials in dem Bereich des Schlitzes oder des Tores (27) mittels einer Schnappassung befestigt zu werden, wobei die Anschlagfläche (28) und/oder der Bereich Rampenflächen (28A) derart aufweisen, daß, wenn sie mittels Schnappassung in den Schlitz oder das Tor (27) eingepaßt ist, die Anschlagfläche (28) innerhalb eines gewählten Schlitzes oder Teiles des Tores (27) derart verriegelt ist, daß eine Bewegungsbegrenzung definiert wird.

9. Spritze nach Anspruch 8,
dadurch **gekennzeichnet,** daß
die Anschlagfläche (28) ein Vorsprung von der Trommel oder dem Schieber (7) ist, der ein Profil mit einer Keiloberfläche, die ermöglicht, daß sich der Vorsprung (28) über die Oberfläche des Schiebers bzw. der Trommel (20) bewegt, wenn die Spritze zusammengesetzt wird, und daß er verriegelnd in seine Stelle in dem gewählten Schlitz oder Teil des Tores (27) einschnappt, und entgegengesetzt zu der Keilfläche (28A) eine Anstoßfläche (28B) aufweist, um zu verhindern, daß die Anschlagfläche (28) nachfolgend entfernt wird.

10. Spritze nach einem der Ansprüche 1 bis 6,
dadurch **gekennzeichnet,** daß
der Einsatz (12) einen Schlüssel aufweist, der in nur eine der zwei oder mehr Stellen (11) in der Trommel (1) oder dem Schieber (7) passen kann.

## Revendications

1. Seringue convenant pour l'administration de liquides, qui comprend un corps (1) qui définit ou comprend un cylindre (2), le cylindre (2) ayant un ajutage (3) à une extrémité et incluant un piston (4) qui peut coulisser longitudinalement à l'intérieur du cylindre (2), la distance dont le piston (4) peut se déplacer à l'intérieur du cylindre (2) définissant le volume qui est administré par la seringue, le piston (4) étant fonctionnellement couplé à un coulisseau (7) qui est agencé de manière à se déplacer en parallèle avec le piston (4), la distance longitudinale dont le piston (4) peut coulisser à l'intérieur du cylindre (2) étant définie par les limites de déplacement d'une surface d'arrêt (12), prévue sur le corps (1) ou le coulisseau (7), par rapport à des butées de fin de course (10A,10B) prévues respectivement sur le coulisseau (7) ou le corps (1),
caractérisée en ce que :
la seringue est verrouillable dans un état dans lequel la surface d'arrêt (12) et les butées de fin de course (10A,10B) peuvent seulement se déplacer les unes par rapport aux autres dans des limites de déplacement choisies parmi deux limites de déplacement prédéterminées sélectables, ou davantage, et
la surface d'arrêt (12) comprend un insert (12A, 12B) qui est insérable dans un trou (11) prévu dans le corps (1) ou le coulisseau (7) de manière à faire saillie hors du trou et à pouvoir être enlevé du trou seulement avec difficulté ou au moyen d'un outil spécial, la partie saillante(12B) de l'insert formant une surface d'arrêt (12), la position de chaque dit trou (11) par rapport aux butées de fin de course (10A,10B) définissant une des distances de déplacement présélectionnées.

2. Seringue selon la revendication 1, caractérisée en ce que les limites de déplacement présélectionnables sont définies par les limites de déplacement d'une surface d'arrêt (12),prévue sur le corps (1) ou le coulisseau (7), entre des butées de fin de course (10A,10B) définies comme étant les extrémités d'une rainure sensiblement longitudinale (10) prévue sur le coulisseau (7) ou le corps (1) respectivement.

3. Seringue selon la revendication 2,
caractérisée en ce que la seringue comporte deux dites rainures (10) ou davantage, qui ont des longueurs différentes correspondant aux deux limites de déplacement présélectables ou davantage, ou bien la rainure (10) est définie par une ouverture (14) ayant une configuration à rampe ou à gradins, chaque partie de la rampe ou chaque gradin définissant une longueur de rainure différente, ou bien la rainure est sous la forme d'une ouverture (18) à l'intérieur de laquelle on peut choisir la position de la surface d'arrêt (16) par rapport aux extrémités de la rainure.

4. Seringue selon la revendication 1,
caractérisée en ce que le coulisseau (7) est sous la forme d'un manchon entourant complètement ou partiellement le corps (1), le manchon comporte deux trous (11) ou davantage qui traversent complètement sa paroi, chaque trou (11) étant adjacent à une rainure (10) ou une ouverture (14) formée dans où à travers la surface du corps (1), chaque trou (11) définissant une position sélectable de l'insert (12) par rapport aux extrémités de la rainure (10) ou de l'ouverture (14), de façon à définir différentes limites de déplacement.

5. Seringue selon la revendication 1,
caractérisée en ce que le coulisseau (7) est sous la forme d'un manchon entourant complètement ou partiellement le corps (1), le manchon (7) comportant deux rainures (17) ou davantage, chaque rainure (17) ayant une longueur différente, ou bien une ouverture (18) traversant complètement sa paroi, la région correspondante de la surface du corps (1) comportant un ou plusieurs trous (15), chaque trou (15) étant adjacent à une rainure (17) ou à l'ouverture (18), chaque trou (15) définissant une position sélectable de l'insert (16) par rapport aux extrémités de la rainure (17) ou de l'ouverture (18), de façon à définir des limites de déplacement différentes.

6. Seringue convenant pour l'administration de liquides, comprenant un corps (20) qui définit ou comprend un cylindre (19), le cylindre (19) ayant un ajutage (3) à une extrémité et incluant un piston (4) qui peut coulisser longitudinalement à l'intérieur du cylindre (19), la distance dont le piston (4) peut se déplacer à l'intérieur du cylindre (19) définissant le volume qui est administré par la seringue, le piston (4) étant fonctionnellement couplé à un coulisseau (7) qui est agencé de manière à se déplacer en parallèle avec le piston (4), la distance longitudinale dont le piston (4) peut coulisser à l'intérieur du cylindre (19) étant définie par les limites de déplacement d'une surface d'arrêt (22),prévue sur le corps (1) ou le coulisseau (7), par rapport à des butées de fin de course (23A,23B) prévues sur le coulisseau (7) ou le corps (1) respectivement,
caractérisée en ce que :
la seringue est verrouillable dans un état dans lequel la surface d'arrêt (22) et les butées de fin de course (23A,23B) peuvent seulement se déplacer les unes par rapport aux autres dans des limites de déplacement choisies parmi deux limites de déplacement sélectables prédéterminées ou davantage, le coulisseau (7) étant sous la forme d'un manchon qui entoure le cylindre (19) et est lui-même entouré par le corps (20), et qui peut tourner par rapport au corps (20) et coulisser longitudinalement par rapport au cylindre (19), et la rotation relative du coulisseau (7) et du corps (19) modifie les positions relatives de la surface d'arrêt (22) et des butées de fin de course (23A,23B) de façon à définir différentes limites de déplacement de la surface d'arrêt à l'intérieur d'une ou plusieurs rainures ou d'une ouverture (23), la ou les rainures ou l'ouverture (23) étant déplaçables par rapport à une surface d'arrêt fixe (22) prévue sur le corps (20) ou le coulisseau (7), et le coulisseau (7) et le corps (20) sont verrouillables en position au moyen d'un insert de verrouillage (25) qui est insérable dans un trou (24) traversant la paroi du corps (20), de manière à pénétrer dans un trou correspondant du coulisseau (7),et qui peut en être retiré seulement avec difficulté ou au moyen d'un outil spécial.

7. Seringue selon la revendication 6,
caractérisée en ce que le corps (20) comporte une ouverture à gradins (23) traversant complètement sa paroi, une saillie (22) étant prévue sur la surface du coulisseau (7) à partir de la région du coulisseau (7) adjacente à l'ouverture (23), et la rotation relative du corps (20) sur le coulisseau (7) amène la saillie (22) en position alignée avec des parties de l'ouverture (23) ayant des longueurs différentes, de façon à définir des limites de déplacement différentes.

8. Seringue convenant pour l'administration de liquides, comprenant un corps (20) qui définit ou comprend un cylindre (19), le cylindre (19) ayant un ajutage (3) à une extrémité et incluant un piston (4) qui peut coulisser longitudinalement à l'intérieur du cylindre (19),la distance dont le piston (4) peut se déplacer à l'intérieur du cylindre (19) définissant le volume qui est administré par la seringue, le piston (4) étant fonctionnellement couplé à un coulisseau (7) qui est agencé de manière à se déplacer en parallèle avec le piston (4), la distance longitudinale dont le piston (4) peut coulisser à l'intérieur du cylindre (19) étant définie par les limites de déplacement d'une surface d'arrêt (28), prévue sur le corps (20) ou le coulisseau (7), par rapport à des butées de fin de course (27A,27B) prévues sur le coulisseau (7) ou le corps (20) respectivement, les butées de fin de course (27A,27B) étant définies comme étant les limites de déplacement de la surface d'arrêt (28) dans une ouverture ou une rainure (27),
caractérisée en ce que :
la seringue est verrouillable dans un état dans lequel la surface d'arrêt (28) et les butées de fin de course (27A,27B) peuvent seulement se déplacer les unes par rapport aux autres dans des limites de déplacement choisies parmi deux limites de déplacement sélectables prédéterminées ou davantage, l'ouverture ou la ou les rainures (27) et la surface d'arrêt (28) étant déplaçables les unes par rapport aux autres, et la surface d'arrêt (28) pouvant être enclenchée élastiquement en position dans la rainure ou l'ouverture (27) contre l'élasticité de la matière de la seringue dans la région de la rainure ou de l'ouverture (27), la surface d'arrêt (28) et/ou la région présentant des surfaces inclinées (28A) de sorte que, lors de l'enclenchement élastique dans la rainure ou l'ouverture (27), la surface d'arrêt (28) est bloquée dans une rainure ou une partie de l'ouverture (27) choisie pour définir une limite de déplacement.

9. Seringue selon la revendication 8,
caractérisée en ce que la surface d'arrêt (28) est une saillie formée sur le corps ou le coulisseau (7), ayant un profil en coin qui permet à la saillie (28) de franchir la surface du coulisseau ou du corps (20) respectivement lors de l'assemblage de la seringue et de s'enclencher en position de verrouillage dans la rainure ou la partie de l'ouverture (27) choisie, et présentant une face de butée (28B) opposée à la face en coin (28A) pour empêcher ensuite l'enlèvement de la surface d'arrêt (28).

10. Seringue selon la revendication 1 ou 6,
caractérisée en ce que l'insert (12) comprend une clé qui peut s'ajuster seulement dans une de deux positions (11) ou davantage, dans le corps (1) ou le coulisseau (7).
